# EUROPEAN PATENT SPECIFICATION

(11) **EP 1 251 175 B1**
(45) Date of publication and mention of the grant of the patent: **08.11.2006**
(21) Application number: 02290998.0
(22) Date of filing: 19.04.2002
(51) Int. Cl.: C12N 15/52, C12N 15/10, C12N 9/00, C12N 1/21, C12P 21/02

(54) **Method for diversifying the chemical composition of protein produced in vivo by genetically disabling the editing function of their aminoacyl tRNA synthetases**
Methode für die Erweiterung der chemischen Zusammensetzung von Proteinen produziert in vivo unter Verwendung von mutierten Aminoacyl-tRNA-Synthetasen ohne Korrekturfunktion
Procédé de production de protéines comprenant des acides aminés non conventionnels utilisant des aminoacyl-tRNA synthétases mutantes sans fonction de correction

(30) Priority: 19.04.2001 US 285495 P
(43) Date of publication of application: 23.10.2002
(73) Proprietor: INSTITUT PASTEUR, 75015 Paris (FR); Evologic GmbH, 50677 Köln (DE); THE SCRIPPS RESEARCH INSTITUTE, La Jolla, CA 92037 (US)
(72) Inventor: Doring, Volker, 75015 Paris (FR); Nangle, Leslie A., San Diego, CA 92110 (US); Hendrickson, Tamara L., Baltimore, MD 21217 (US); de Crecy-Lagard, Valérie, La Jolla, CA 92037 (US); Schimmel, Paul, La Jolla, CA 92037 (US); Marlière, Philippe, 75012 PARIS (FR)
(74) Representative: Santarelli

(56) References cited:
- WO-A-00/24922
- WO-A-01/83718
- US-A- 5 370 995
- DORING VOLKER ET AL: "Enlarging the amino acid set of Escherichia coli by infiltration of the valine coding pathway." SCIENCE (WASHINGTON D C), vol. 292, no. 5516, 20 April 2001 (2001-04-20), pages 501-504, XP002207263 ISSN: 0036-8075
- BRUNNER J: "BIOSYNTHETIC INCORPORATION OF NON-NATURAL AMINO ACIDS INTO PROTEINS" CHEMICAL SOCIETY REVIEWS, CHEMICAL SOCIETY, LONDON, GB, vol. 22, no. 3, 1993, pages 183-189, XP000978937 ISSN: 0306-0012
- LEMEIGNAN B ET AL: "Phenotypic suppression by incorporation of an alien amino acid" JOURNAL OF MOLECULAR BIOLOGY, LONDON, GB, vol. 231, no. 2, May 1993 (1993-05), pages 161-166, XP002113371 ISSN: 0022-2836
- IBBA ET AL: "Towards engineering proteins by site-directed incorporation in vivo of non-natural amino acids" BIO/TECHNOLOGY, NATURE PUBLISHING CO. NEW YORK, US, vol. 12, no. 7, July 1994 (1994-07), pages 678-682, XP002158041 ISSN: 0733-222X
- LIU D R ET AL: "PROGRESS TOWARD THE EVOLUTION OF AN ORGANISM WITH AN EXPANDED GENETIC CODE" PROCEEDINGS OF THE NATIONAL ACADEMY OF SCIENCES OF USA, NATIONAL ACADEMY OF SCIENCE. WASHINGTON, US, no. 96, April 1999 (1999-04), pages 4780-4785, XP001040352 ISSN: 0027-8424

### SEQUENCE LISTING

<110> INSTITUT PASTEUR
   THE SCRIPPS RESEARCH INSTITUTE
   EVOLOGIC GmbH
<120> Method for diversifying the chemical composition of proteins produced in-vivo by genetically disabling the editing function of their aminoacyl tRNA synthetase
<130> BIF 023431 EP
<140> EP/02290998
   <141> 2002-04-19
<150> US 60/285,495
   <151> 2001-04-19
<160> 7
<170> PatentIn version 3.1
<210> 1
   <211> 52
   <212> DNA
   <213> artificial
<220>
   <221> primer_bind
   <222> (1)..(52)
   <223> 5' phosphorylated oligonucleotide for introducing mutation in the editing domain of isoleucyl-tRNA synthetase gene Ile RS
<400> 1
   ctagtaatcg cggcggcggc ggcggcggcg gcggcggcgg cgcgcgcaat at 52
<210> 2
   <211> 50
<212> DNA
   <213> artificial
<220>
   <221> misc_binding
   <222> (1)..(50)
   <223> 5' phosphorylated oligonucleotide for introducing mutation in the editing domain of isoleucyl-tRNA synthetase gene Ile RS
<400> 2
   cgatattgcg cgcgccgccg ccgccgccgc cgccgccgcc gccgcgatta 50
<210> 3
   <211> 36
   <212> DNA
   <213> artificial
<220>
   <221> misc_binding
   <222> (1)..(36)
   <223> 5' phosphorylated oligonucleotide for introducing mutation in the thyA gene
<400> 3
   ctggataaaa tggcgctagc accgtgccat gcattc 36
<210> 4
   <211> 36
   <212> DNA
   <213> artificial
<220>
   <221> misc_binding
   <222> (1)..(33)
   <223> 5' phosphorylated oligonucleotide for introducing mutation in thy A gene
<400> 4
   tctgccacat agaactggaa gaatgcatgg cacggt 36
<210> 5
   <211> 33
   <212> DNA
   <213> artificial
<220>
   <221> misc_binding
   <222> (1)..(33)
   <223> 5' phosphorylated PCR oligonucleotide to amplify vals gene
<400> 5
   ggggaattcg gtgtgtgaaa ttgccgcaga acg 33
<210> 6
   <211> 33
   <212> DNA
   <213> artificial
<220>
   <221> misc_binding
   <222> (1)..(33)
   <223> 5' phosphorylated PCR oligonucleotide to amplify vals gene
<400> 6
   ggcaagcttt caggtatttg ctgcccagat cga 33
<210> 7
   <211> 17
   <212> PRT
   <213> artificial
<220>
   <221> misc_feature
   <223> AlaXp yeast protein fragment (Lys156-Arg172 swissprot:p53960)
<400> 7
   Lys Pro Val Leu Glu Pro Ser Asp Tyr Phe Asn Tyr Ile Glu Leu Asn Arg
   1               5                   10                  15

## Description

### BACKGROUND OF THE INVENTION

The present invention is directed to a method to diversify the chemical composition of proteins produced *in vivo* comprising the step of disabling, particularly by mutagenesis, the editing function of one of its aminoacyl tRNA synthetases. The present invention is also directed to nucleic acid sequences encoding such mutated aminoacyl tRNA synthetases having their editing site mutated and capable of mischarging its cognate tRNA with a noncanonical amino acid. Also described herein is an improved method for obtaining transformed cells capable of synthetizing *in vivo* proteins comprising at least a noncanonical amino acid and their use for the production of such proteins.

Aminoacyl tRNA synthetases establish the rules of the genetic code by catalyzing the aminoacylation of transfer RNAs. The chemical invariance of the twenty amino acid building blocks of proteins is well established. The only known extensions to this invariant set are formyl-methionine (1) and selenocysteine (2), both incorporated in response to punctuation signals during translation in certain organisms. Thus, although species have colonized dissimilar terrestrial habitats throughout geological times, this diversification has not been mirrored in the evolution of organisms to include specialized sets of amino acids. For instance, thermophilic, mesophilic, and psychrophilic organisms all assemble proteins by combining the same types of twenty canonical amino acids into different protein sequences. Standing as the "missing link" between alanine and valine (3), aminobutyrate (Abu, also known as butyrine) can be generated by transamination from the physiological metabolite 2-oxo-butyrate and should thus be considered as a latent metabolite (4). Its absence is therefore particularly conspicuous in the proteins of extant organisms.

The selection of amino acids for protein synthesis is done by aminoacyl tRNA synthetases. Typically, each of twenty synthetases catalyzes the attachment of its cognate amino acid to the 3'-end of its cognate tRNA and amino acids are, in this way, associated with specific triplets of the genetic code (5). The active site of several of these enzymes inherently lack the capacity to discriminate between closely similar amino acids at a level sufficient to explain the high accuracy of the code. For that reason, a given enzyme may misactivate closely similar (in size and shape) amino acids at a low frequency (0.1 to 1 %) (6). To correct these errors, in many cases, a hydrolytic editing function, at a separate active site, has developed (7-10). One example of a synthetase that has editing activity is valyl-tRNA synthetase (VaIRS), which misactivates the isosteric natural amino acid Thr (9), as well as the non-natural Abu (11). Misactivation of these amino acids leads to transient mischarging of tRNA^{Val}, followed by hydrolytic deacylation (editing) of the mischarged amino acid from the tRNA.

The present work aimed to establish conditions of artificial selection that promoted usage of non-canonical amino acids, such as Abu, that were not retained by natural selection. Others have attempted to incorporate a non-canonical amino acid into a protein by introducing a foreign, "orthogonal" tRNA/synthetase pair that can insert the amino acid at a specialized stop codon (12).

However, such approaches are laborious, as they require selection, identification, cloning, and study of individual mutant strains.

In order to facilitate the *in vivo* production of proteins comprising noncanonical amino acids, it would be desirable to have a rapid and generalized method allowing to genetically modify and select cells capable of achieving the *in vivo* production of such proteins.

Such a desirable method will allow to enlarge the chemistry of translation by having a non-canonical amino acid "infiltrate" all of the codons normally associated with one of the natural amino acids. Indeed, by assigning two amino acids (a cognate and a noncognate) to a specific set of codons so as to provide a selective advantage to the reprogrammed cells, global changes in the amino acid compositions of all cellular proteins could be made.

This is the object of the present invention.

### SUMMARY OF THE INVENTION

It has been developed a general method to diversify the chemical composition of proteins produced *in vivo* by a cell comprising the step wherein the editing function of one of an aminoacyl tRNA synthetase of said cell has been disabled.

This rapid and general method may be used to obtain cells comprising a mutation in the DNA sequence encoding the editon domain of said disabled aminoacyl tRNA synthetase compared to the wild type aminoacyl tRNA synthetase coding sequence.

In general, the method described herein includes: a) selecting a cell strain wherein the editing function of one of its aminoacyl tRNA synthetases has been disabled, said disabled editing function allowing the aminoacyl tRNA synthetase to mischarge the cognate tRNA with said at least one noncanonical amino acid; b) culturing the selected strain in a culture medium comprising said noncanonical amino acid, or one of its precursor, under conditions favourable for the growth of said strain; and c) recovering from the culture medium or from the cells obtained in step b) the proteins containing said noncanonical amino acid.

In various examples, the editing function of the aminoacyl tRNA synthetases has been disabled by mutagenizing the DNA sequence encoding the editing domain of an aminoacyl tRNA synthetase in the target cell, said mutagenesis being carried out in the cell preferably by homologous recombination or allele replacement vector leading to an aminoacyl tRNA synthetase variant having an amino acid mutation in its editing domain, said mutation allowing the aminoacyl tRNA synthetase variant to mischarge its cognate tRNA with one noncanonical amino acid.

Also disclosed herein is a method for selecting an aminoacyl tRNA synthetase variant capable of mischarging its cognate tRNA with a noncognate amino acid, preferably a noncanonical amino acid, including the steps of: a) elaborating a DNA construct encoding an aminoacyl tRNA synthetase variant having an amino acid mutation in its editing domain; b) transforming a host cell with said DNA construct; c) assaying the ability of the recombinant aminoacyl tRNA synthetase variant produced by said transformed host cell for its ability to mischarge its cognate tRNA with a noncognate amino acid, preferably a noncanonical amino acid; and d) if appropriate, selecting the assayed aminoacyl tRNA synthetase variant if said assayed aminoacyl tRNA synthetase variant is capable of mischarging its cognate tRNA (tRNA(s) associated with the assayed aminoacyl tRNA synthetase) with a noncognate amino acid, preferably with a noncanonical amino acid.

The invention relates to isolated isoleucyl tRNA synthetase variants capable of mischarging its cognate tRNA with a noncognate amino acid, preferably with a noncanonical amino acid, wherein the nucleic fragment encoding the editing site comprises at least one mutation leading to an amino acid mutation, preferably an amino acid substitution, in the editing site of said isoleucyl tRNA synthetase. Isolated nucleic acid encoding such isoleucyl tRNA synthetase variants, vectors, such as plasmid, and cell comprising such nucleic acid also form part of the present invention.

In another preferred embodiment, the present invention is directed to a method for the production of proteins comprising a noncanonical amino acid including the general steps of: a) culturing, in a culture medium containing said noncanonical amino acid, or one of its precursors, a transformed host cell comprising an isoleucyl tRNA synthetase allele variant capable of mischarging its cognate tRNA with said noncanonical amino acid ; and b) recovering and, if appropriate, purifying the proteins comprising said noncanonical amino acid from the culture medium (supernatant) and/or from the cells (from cells pellet) of step a).

The invention allows the production of proteins comprising a noncanonical amino acid by the above method.

Other features and advantages of the invention will be apparent from the following description of the preferred embodiments thereof, and from the claims.

### BRIEF DESCRIPTION OF THE DRAWINGS

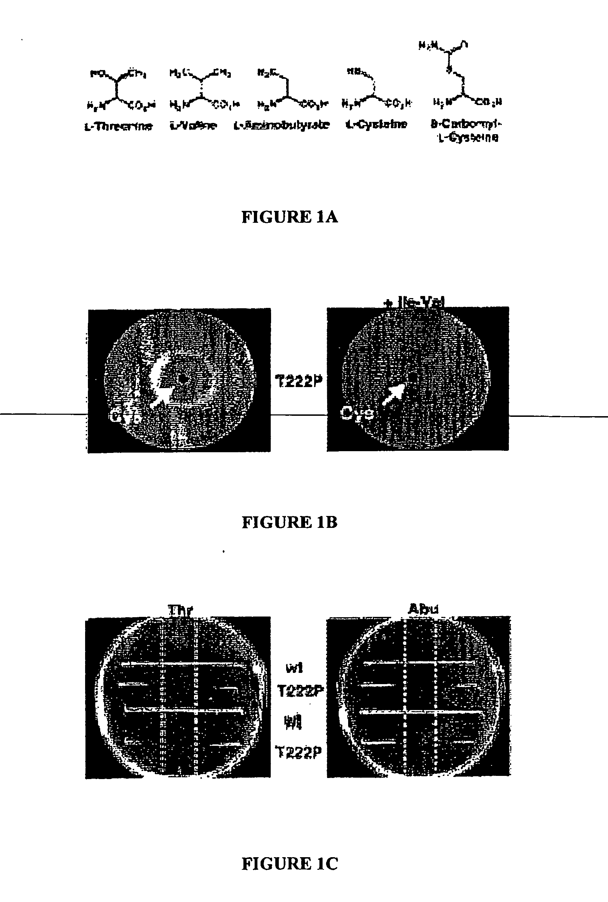

**Figures 1A to 1C.** Suppression and toxicity phenotypes. Amino acid gradient plates were prepared using minimal medium (27) (See also figures 1A to 1C in Doring et al., Science, 292(5516), 453-454, 2001, which are identical) .
**Figure 1A:** Structures of cysteine, S-carbamoyl-cysteine, valine, threonine and α-aminobutyric acid.
**Figure 1B:** Cysteine (100 µl (0.4 M)) was loaded in a central well after spreading and drying 0.5 ml of a 5/1000 dilution of an overnight culture (in MS glucose medium containing thymidine (0.3 mM)) of β5456 (*thyA::erm*+ *ΔnrdD::kan*+ *valS:T222P* pTS13 (*bla+ thyA*:146GUA)). Ile-Val (0.3 mM) was added in one plate as a control. The dipeptide Ile-Val was purchased from Bachem AG (Bubendorf, Switzerland). Plates were incubated for 2 days at 30°C.
**Figure 1C:** Minimal medium plates supplemented with thymidine (0.3 mM) were pretreated with amino acid solutions by streaking either with Thr (50µl (0.2 M)) or Abu (50 µl (0.1 M)) vertically along the diameter of the plate to create an amino acid gradient. Mutant (β5456) and wild-type (β5419 (*thyA::erm*+ *ΔnrdD::kan*+ pTS13 (*bla*+ *thyA*:146GUA)) strains were then streaked horizontally across the plates and incubated for 2 days at 37°C.

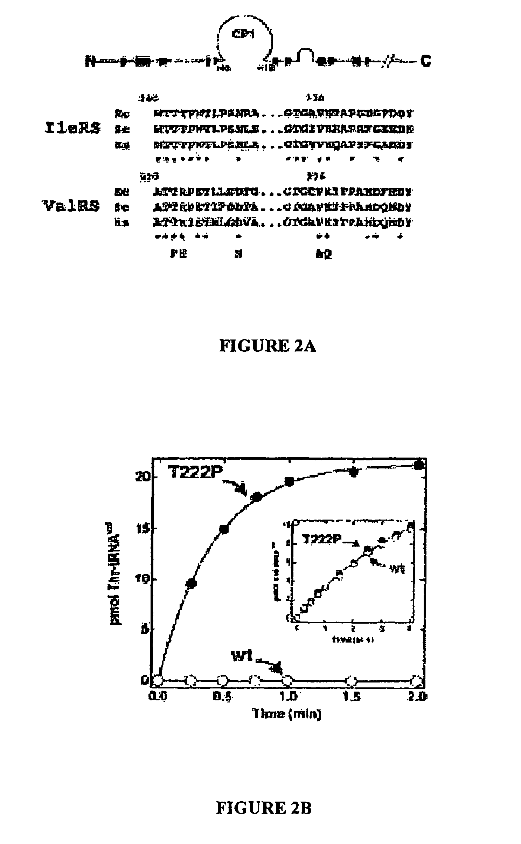

**Figures 2A to 2C.** Point mutations in the editing site and their consequences(See also figures 2A to 2C in Doring et al., Science, 292(5516), 453-454, 2001, which are identical).
**Figure 2A:** Positions of the five point mutations isolated in the editing site of VaIRS are shown. The IleRS editing site (CP1) (28, 29) that intersects the alternating β-strands (pentagons) and α-helices (rectangles) of the catalytic domain is shown. Alignment of residues in the editing sites of HeRS and ValRS is also shown, with the strictly conserved residues among all published sequences labeled with a colon. Abbreviations are Ec, *Escherichia coli*; Sc, *Saccharomyces cerivisiae*; Hs, *Homo sapiens.*
**Figure 2B:** Misaminoacylation of tRNA^{Val} with Thr by the T222P mutant enzyme at pH 7.5 and 37°C. The wild-type (WT) and mutant alleles were cloned under the control of a P_{BAD} promoter (30). The enzymes were partially purified from a laboratory strain lacking the chromosomal copy of the *valS* gene (*ΔvalS::kan*+). The purification and aminoacylation procedures were adapted from Hendrickson *et al.* (31). (Main panel) Misaminoacylation of tRNA^{Val} with Thr by the two enzymes. (Inset) Aminoacylation of tRNA^{Val} with Val by the two enzymes.

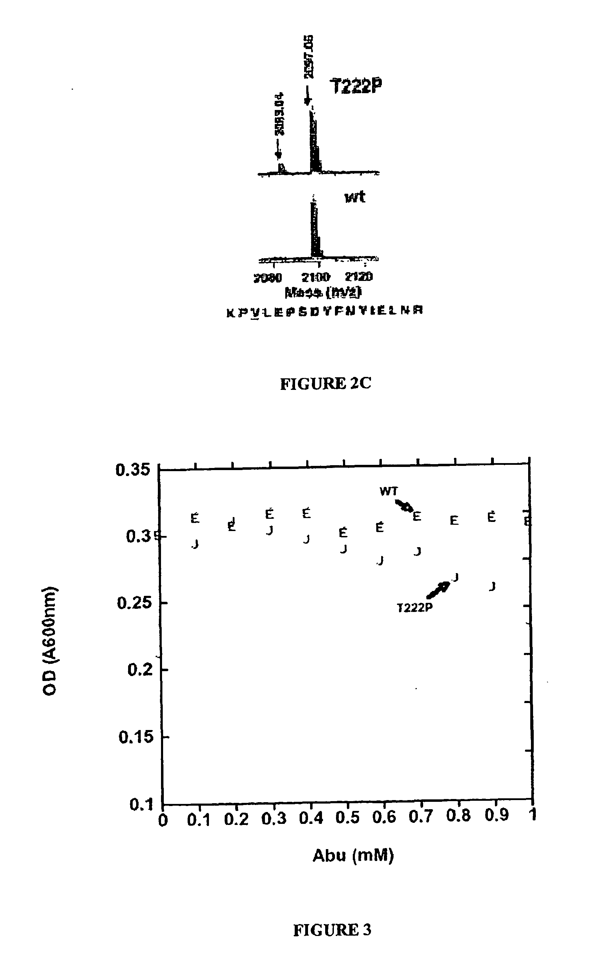

**Figure 2C:** *In vivo* incorporation of aminobutyrate. The His-tagged protein AlaXp was expressed in two *Δilv* strains containing the wild-type *valS* or the mutant *valS:T222P* allele, in MS medium containing Ile-Leu (0.3 mM), Ile-Val (0.02 mM) and Abu (0.2 mM). AlaXp was purified with Ni-NTA agarose (Qiagen GmbH, Hilden, Germany), cut out of a SDS-PAGE preparative gel and prepared for MALDI and µ-LC-MS/MS mass analysis (32). The MALDI-MS analyses were performed in a Voyager-Elite time-of-flight mass spectrometer with delayed extraction (PerSeptive Biosystems, Inc., Framingham, MA). The spectrum for peptide Lys156-Arg172 with mass 2097.04 is shown on the bottom panel (wild-type cells). The top panel shows the peptide resolved into two components when isolated from cells bearing the **T222P** allele of the gene for ValRS. The second component has a mass of 2083.04, exactly 14 mass units less then the "wild-type peptide". Multiple peaks correspond to ¹³C isotopic forms that separate peptides differing by 1, 2, 3, etc. mass units.
**Figure 3.** Overnight cultures of *Δilv* auxotrophs containing the WT *valS* (É) or the mutant *valS:T222P* allele (J) and grown in MS glucose medium with limiting valine (0.04 mM Ile-Val. 0.3 mM Ile-Leu) were diluted 1/1, the Val concentration was adjusted to 0.02 mM and the biomass was determined by measuring the optical density at 600 nm after 24 h of growth at 30 °C.

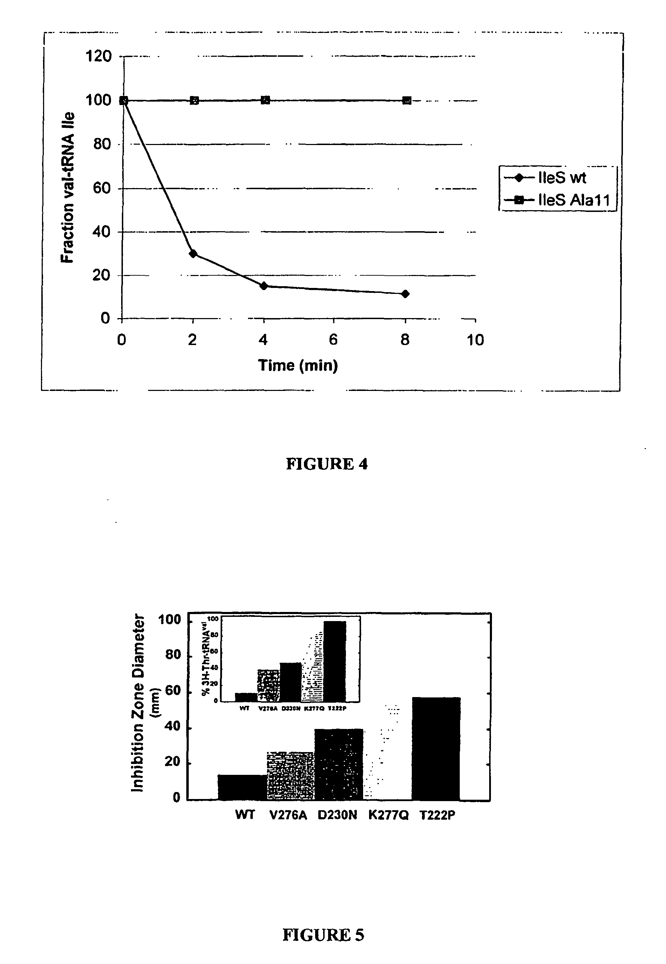

**Figure 4.** Hydrolysis of valyl-tRNAile by wild type and mutated IleRS

This figure shows the large decrease of the editing activity of the mutant IleRS having 11 alanine residues in position aa 240 - 250 compared with the editing activity of the wild type IleRS. ■ :IleS ala 11 (mutant IleRS having 11 alanine residues in position aa 240 - 250); ◆: IleS wt (wild type IleRS)
**Figure 5.** Correlation between Abu toxicity as measured as levels of misaminoacylated [³H]Thr-tRNAᵥₐₗ and toxicity of ABU measured in the different mutant strains. Correlation of Abu toxicity phenotypes and effectiveness of deacylation by mutant enzymes. A. Noncognate amino acid toxicity phenotypes. valS null strains harboring each of the ValRS mutant enzymes in trans were plated on minimal medium. Abu (α-aminobutyrate) was loaded into a central well and plates were incubated for 24 hours at 42°C. Degree of toxicity in response to Abu was evaluated by the diameter of the region of inhibited cell growth. Strains bearing the K277Q and T222P mutant valS alleles showed the most severe response to Abu. The growth of D230N valS was inhibited by high concentrations of Abu but the effect was not observed at lower concentrations. The V276A mutation introduces a slight sensitivity to Abu. Similar phenotypes were observed in response to exogenous threonine (data not shown). B. Histogram representation of the inhibition zone diameters of DvalS::kanR strains harboring each of the ValRS editing mutants on plasmid in response to exogenous Abu. Effects range from mild to severe. (Inset) Histogram representation of the percentage of mischarged Thr-tRNAVal remaining after incubation with 2 nM enzyme for 15 minutes at room temperature. Both show a distinct range in the severity of editing defects caused by these point mutations. Taken together this illustrates a correlation between the ability of the mutant enzyme to deacylate mischarged amino acids from tRNAVal in vitro and the degree of in vivo toxicity observed in response to exogenous noncognate amino acids.

### DETAILED DESCRIPTION OF THE INVENTION

The present invention provides a method to diversify the chemical composition of proteins produced *in vivo* by a cell comprising the step of disabling the editing function of one of its aminoacyl tRNA synthetases.

By the phrase "a step of disabling the editing function of an aminoacyl tRNA synthetase", it is intended to designate a step through which a mutant strain is obtained wherein an allele coding for an aminoacyl tRNA synthetasecomprises a mutation in its editing site or domain resulting to:
- the loss of the role of the editing function in restricting the genetic code to 20 amino acids;
- the loss of the role of the editing function in preventing the invasion of noncognate amino acid; and/or
- the misactivation of noncognate amino acids or/and the non hydrolyzation of generated "noncognate amino acid-tRNA" normally hydrolyzed by the editing function of the wild type cognate aminoacyl tRNA synthetase, these alterations of the editing function inducing the potential misincorporation of a noncognate amino acid, preferably a noncanonical amino acid, in place of the cognate amino acid charged by the aminoacyl tRNA synthetase encoded by the wild type allele.

For example, such a step of disabling the editing function of one of an aminoacyl tRNA synthetase may comprise a step of:
- selecting a mutant strain comprising an aminoacyl tRNA synthetase natural variant having a mutation in its editing domain resulting to these above cited editing function alterations; or preferably
- selecting a mutant strain comprising an aminoacyl tRNA synthetase variant having a mutation in its editing domain resulting to these above cited editing function alterations, this variant being obtained by mutagenesis, such as by homologous recombination or replacement allele vector or by any mutagenesis methods known by the skilled person capable of introducing such a mutation, preferably integrated into the genome of the cell.

By the terms "cognate amino acid" in the present specification, it is intented to designate the amino acid normally charged by the tRNA corresponding to (or associated with) the aminoacyl tRNA synthetase wild type (for example: the cognate amino acid for valyl-tRNA synthetase is valine).

By the terms "cognate tRNA" in the present specification, it is intented to designate the tRNA corresponding to (or associated with) the aminoacyl tRNA synthetase wild type (for example: the cognate tRNA for valyl-tRNA synthetase is tRNA^{Val}).

Further there is provided a method for producing *in vivo* proteins comprising at least one noncanonical amino acid comprising the step of:
a) selecting a cell strain wherein the editing function of one of its aminoacyl tRNA synthetases has been disabled by mutagenesis, said disabled editing function allowing the aminoacyl tRNA synthetase to mischarge the cognate tRNA with said at least one noncanonical amino acid;
b) culturing the selected strain in a culture medium comprising said noncanonical amino acid, or one of its precursor, under favourable conditions for the growth of said strain; and
c) recovering from the culture medium or from the cells obtained in step b) the proteins containing said noncanonical amino acid.

By "favourable growth conditions" is meant an environment that is relatively favorable for cell growth and/or viability. Such conditions take into account the relative availability of nutrients and optimal temperature, atmospheric pressure, presence or absence of gases (such as oxygen and carbon dioxide), and exposure to light, as required by the organism being studied.

By "precursor " is meant a compound which can be efficiently converted *in vivo* by the cell in said noncanonical amino acid.

In a preferred embodiment, the cell strain wherein the editing function of its isoleucyl tRNA synthetases has been disabled, comprises a mutation in the DNA sequence encoding the editing domain of said disabled isoleucyl tRNA synthetase compared to the wild type isoleucyl tRNA synthetase coding sequence.

In a more preferred embodiment, said DNA mutation leads to a replacement of the residues 240 to 250 of the editing domain of the protein encoded by the ile RS gene of E. coli by 11 successive alanine residues.

In another preferred embodiment is provided a method according to the present invention, wherein the disabled isoleucyl tRNA synthetase is capable of mischarging its cognate tRNA with a canonical amino acid sterically similar to the amino acid charged by the wild type isoleucyl tRNA synthetase on its cognate tRNA.

In another preferred embodiment is provided a method according to the present invention, wherein the disabled isoleucyl tRNA synthetase is capable of mischarging its cognate tRNA with a noncanonical amino acid sterically similar to the amino acid charged by the wild type isoleucyl tRNA synthetase on its cognate tRNA.

In another aspect of the present invention, there is provided a method for obtaining cells capable of producing *in vivo* proteins comprising at least one noncanonical amino acid comprising the step of mutagenizing the DNA sequence encoding the editing domain of an l isoleucyl tRNA synthetase in a cell, said mutagenizing leading to an isoleucyl tRNA synthetase variant having an amino acid mutation in its editing domain and said mutation allowing the isoleucyl tRNA synthetase variant to mischarge its cognate tRNA with said at least one noncanonical amino acid.

There is disclosed a method for obtaining cells capable of producing *in vivo* proteins comprising at least one noncanonical amino acid according to the present invention, comprising the steps of:
a) assaying the ability of an aminoacyl tRNA synthetase variant mutated in its editing domain for its ability to mischarge its cognate tRNA with a noncanonical amino acid;
b) mutagenizing the DNA sequence encoding the editing domain of said aminoacyl tRNA synthetase in a cell, said mutagenizing leading to replace the allele encoding the wild type aminoacyl tRNA synthetase by an allele encoding the aminoacyl tRNA synthetase variant assayed in step a) and capable of producing detectable noncanonical amino acid mischarging;
c) optionally, identifying, selecting and/or cloning the cells containing such aminoacyl tRNA synthetase variant having the ability to mischarge one noncanonical amino acid.

In a preferred embodiment, the cell wherein the editing domain of the isoleucyl tRNA synthetase has been disabled, preferably by mutagenesis, is a microbial or animal cell, preferably a bacterium, a yeast or a fungus, more preferably a bacterium such as *Escherichia coli* or *Acinetobacter.*

In another preferred embodiment of the present invention, is provided a method, wherein the editing domain of the isoleucyl tRNA synthetase of the target cell has been disabled by homologous recombination or by recombination into the genome of the target cell using an allelic replacement vector.

The oligonucleotides comprising the nucleic fragment encoding the mutated editing site, or portion thereof, and which contain the mutation which have to be introduce in the wild type allele of the target cell, can be chemically synthetized or synthetized by Polymerase Chain Reaction (PCR).

By microbial or animal cells are preferred cells that undergo homologous recombination. Such cells may be of bacterial, mycobacterial, yeast, fungal, algal, plant, or animal origin.

By "homologous recombination" is meant a process by which an exogenously introduced DNA molecule integrates into a target DNA molecule in a region where there is identical or near-identical nucleotide sequence between the two molecules. Homologous recombination is mediated by complementary base-pairing, and may result in either insertion of the exogenous DNA into the target DNA (a single cross-over event), or replacement of the target DNA by the exogenous DNA (a double cross-over event).

By "allelic replacement vector" is meant any DNA element that can be used to introduce mutations into the genome of a target cell by specific replacement of a native gene with a mutated copy. For example, gene replacement in bacteria is commonly performed using plasmids that contain a target gene containing a mutation and a negative selectable marker outside of the region of homology. Such a plasmid integrates into the target chromosome by homologous recombination (single cross-over). Appropriate selection yields cells that have lost the negative selection marker by a second homologous recombination event (double cross-over) and contain only a mutant copy of the target gene.

In another embodiment, the cell wherein the editing domain of the isoleucyl tRNA synthetase has been disabled or mutagenized may contain a selectable marker gene for identifying and selecting the cells containing the mutagenized DNA. The identification and selection of that cells wherein the editing domain of the isoleucyl tRNA synthetase has been disabled or mutagenized may be based upon the ability of the cells to grow on selective medium, wherein a cell containing the selectable marker can grow on selective medium, and a cell lacking this selectable marker cannot grow, or grows more slowly, on selective medium.

In still another embodiment, the cell wherein the editing domain of the isoleucyl tRNA synthetase has been disabled or mutagenized may contain a reporter gene, for identifying and selecting the cells containing the mutagenized DNA. The identification and selection of that cells wherein the editing domain of the isoleucyl tRNA synthetase has isoleucyl been disabled or mutagenized may be based on a reporter gene assay, wherein the expression by the cell of the reporter gene confirms the disabling or the mutagenesis and a cell lacking the disabling or the mutagenesis does not express the reporter gene.

By "selectable marker" is meant a gene that alters the ability of a cell harboring this gene to grow or survive in a given growth environment relative to a similar cell lacking the selectable marker. Such a marker may be a positive or negative selectable marker. For example, a positive selectable marker (e.g., an antibiotic resistance or auxotrophic growth gene) encodes a product that confers growth or survival abilities in selective medium (e.g., containing an antibiotic or lacking an essential nutrient). A negative selectable marker, in contrast, prevents cells harbouring this gene from growing in negative selection medium, when compared to cells not harbouring this gene. A selectable marker may confer both positive and negative selectability, depending upon the medium used to grow the cell. The use of selectable markers in prokaryotic and eukaryotic cells is well known by those of skill in the art.

By "reporter gene" is meant any gene which encodes a product whose expression is detectable and/or quantitatable by immunological, chemical, biochemical, biological, or mechanical assays. A reporter gene product may, for example, have one of the following attributes, without restriction: fluorescence (e.g., green fluorescent protein), enzymatic activity (e.g., lacZ/.beta.-galactosidase, luciferase, chloramphenicol acetyltransferase, alkaline phosphatase), toxicity (e.g., ricin), or an ability to be specifically bound by a second molecule (e.g., biotin or a detectably labelled antibody). It is understood that any engineered variants of reporter genes, which are readily available to one skilled in the art, are also included, without restriction, in the foregoing definition.

By "identifying cells containing mutagenized DNA" is meant exposing the population of cells transformed with the mutagenized DNA to selective pressure (such as growth in the presence of an antibiotic or the absence of a nutrient) consistent with a selectable marker carried by the cell containing the recombined mutagenized DNA (e.g., an antibiotic resistance gene or auxotrophic growth gene known to those skilled in the art). Identifying cells containing the recombined mutagenized DNA may also be done by subjecting transformed cells to a reporter gene assay. Selections and screens may be employed to identify cells containing the recombined mutagenized DNA, although selections are preferred.

There is described herein a method for selecting an aminoacyl tRNA synthetase variant capable of mischarging its cognate tRNA with a noncognate and/or a noncanonical amino acid, comprising the steps of:
a) elaborating a DNA construct comprising a DNA sequence encoding an aminoacyl tRNA synthetase variant wherein said aminoacyl tRNA synthetase variant has at least an amino acid mutation, preferably a substitution, more preferably a single substitution, in its editing domain compared with the wild type aminoacyl tRNA synthetase;
b) transforming a host cell with the DNA construct of step a) and, after a step of culturing said transformed host cell, recovering and, optionally, purifying the recombinant aminoacyl tRNA synthetase variant expressed by the host cell;
c) assaying the ability of the recombinant aminoacyl tRNA synthetase variant recovered in step b) for its ability to mischarge its cognate tRNA with a noncognate and/or a noncanonical amino acid; and
d) selecting said aminoacyl tRNA synthetase variant if detectable mischarging has been produced in step c).

Such methods for assaying the ability of the recombinant aminoacyl tRNA synthetase variant recovered in step b) for its ability to mischarge its cognate tRNA with a noncognate and/or a noncanonical amino acid, are disclosed for example in the following examples (see figure 2B). The methods known to those skilled in the art for assaying such an ability may be used.

By "transformation" is meant any method for introducing foreign molecules, such as DNA, into a cell. Lipofection, DEAE-dextran-mediated transfection, microinjection, protoplast fusion, calcium phosphate precipitation, retroviral delivery, electroporation, natural transformation, and biolistic transformation are just a few of the methods known to those skilled in the art which may be used.

The isolated nucleic acid sequence encoding the isoleucyl tRNA synthetase variant according to the invention or vectors comprising a nucleic acid encoding said isoleucyl tRNA synthetase variant form also part of the present invention.

In another aspect, the invention provides a transformed cell comprising a nucleic acid encoding an isoleucyl tRNA synthetase variant according to the present invention.

In a preferred embodiment, said transformed cells are characterized in that said nucleic acid encoding an isoleucyl tRNA synthetase variant according to the present invention is integrated in the genome of said cell.

In a more preferred embodiment, said transformed cells are characterized in that the nucleic fragment comprising the substitution, leading to the alteration of the editing function of the isoleucyl tRNA synthetase variant, has been integrated into the genome of said transformed cell by using mutagenesis, such as homologous recombination, replacement allele vector or any mutagenesis methods for introducing nucleic acid molecules such as DNA, into a cell, known to those skilled in the art, such as lipofection, DEAE-dextran-mediated transfection, microinjection, protoplast fusion, calcium phosphate precipitation, retroviral delivery, electroporation, natural transformation and biolistic transformation.

The invention also relates to isolated prokaryotic or eukaryotic cells capable of producing a protein the amino acid sequence of which comprises at least one noncanonical amino acid, characterized in that they comprise an isoleucyl tRNA synthetase variant which is capable of charging onto one of its cognate tRNAs a noncanonical amino acid or an amino acid other than the cognate amino acid, and in that the nucleic acid sequence of the allele encoding said isoleucyl tRNA synthetase variant includes a substitution of the residues 240 to 250 of the editing domain by 11 successive alanine residues compared with the sequence of the corresponding wild-type allele, said mutation integrated into the genome being located on the editing site of said isoleucyl tRNA synthetase and having been introduced by a technique of mutagenesis, such as genetic recombination.

In another aspect, the invention also comprises the use of a transformed cell according to the invention for producing protein, in particular recombinant protein, the amino acid sequence of which comprises at least one unconventional amino acid.

In a preferred embodiment, the present invention is directed to a method for the production of proteins comprising a noncanonical amino acid characterized in that said method comprises the following steps:
a) culturing a transformed cell comprising an allele encoding an isoleucyl tRNA synthetase variant according to the invention in a culture medium containing a noncanonical amino acid capable of being mischarged by the cognate tRNA of the isoleucyl tRNA synthetase variant contained in said cell, in a culture medium and under culture conditions which allow the growth of said cell; and
b) recovering and, optionally, purifying the proteins comprising said noncanonical amino acid from the culture medium (supernatant) or from the cells (cells pellet) of step a).

Proteins comprising a noncanonical amino acid can be obtained by the above method according to the invention.

The processes for purifying protein, which may be natural or recombinant, conventionally used by those skilled in the art generally employ methods used individually or in combination, such as fractionation, chromatography methods, immunoaffinity techniques using specific mono- or polyclonal antibodies, etc.

The presence of a noncanonical amino acid on the protein to be purified, which noncanonical amino acid could have a specific functional group, may facilitate its purification by reacting selectively with the purification support without modifying the activity of the protein.

Among the proteins which can be produced by a process according to the invention, mention may be made, but without being limited , of proteins which, through the incorporation of at least one noncanonical amino acid, make it possible to obtain a desired activity which a protein the sequence of which includes only canonical amino acids does not make it possible to obtain. The term "activity" is intended to refer, in general, to any activity such as a physiological or biological activity, even partial, relating to unicellular or multicellular organisms, such as for example a structural or biochemical activity, for example an enzymatic or antigenic activity, an activity of antibody type, or an activity which modulates, regulates or inhibits biological activity, or such that it allows the implementation thereof in a process for biosynthesizing or for biodegrading chemical or biochemical compounds.

Among the proteins which can be produced by a process according to the invention, mention may also be made of proteins for which the incorporation of at least one noncanonical amino acid is carried out such that there results therefrom no substantial modification of the biological activity of the corresponding unmodified protein. Besides the conserved biological activity of the corresponding unmodified protein, these proteins according to the invention will have a noncanonical amino acid with specific properties which may be advantageously exploited.

Among the specific properties conferred by the presence of a noncanonical amino acid, mention may be made in particular of the properties linked to the presence of a functional group on said noncanonical amino acid, capable of reacting easily and specifically with a chemical or biochemical compound under conditions which make it possible not to modify the activity of the protein or which avoid modifying the conventional amino acids.

The presence of this specific functional group may advantageously be used, for example, for:
(i) purifying any protein, in particular any recombinant protein, which incorporates said unconventional amino acid;
(ii) coupling such a protein to a solid support;
(iii) coupling to such a protein molecules capable of being detected, such as spectroscopic probes of varied nature;
(iv) coupling to such a protein lipophilic or hydrophilic polymers which allow the solubilization thereof in solvents or which allow masking against recognition by antibodies;
(v) coupling such a protein to a polynucleotide;
(vi) coupling such a protein to a chemical or biochemical compound the presence of which makes it possible to increase, to decrease, to modify, to regulate or to target the biological activity of said protein, or to modify the bioavailability thereof as a compound for therapeutic use; or
(vii)permanently attaching to such a protein a coenzyme which otherwise would diffuse in solution.

Also included in the present invention are the processes according to the invention, characterized in that said transformed cell according to the present invention comprises a homologous or heterologous gene of interest the coding sequence of which includes at least one codon encoding an amino acid the cognate tRNA of which can be mischarged by the isoleucyl tRNAs synthetase variant contained in said transformed cell.

In general, the homologous or heterologous gene of interest, which can be isolated by any conventional technique, such as cloning or PCR (Polymerase Chain Reaction), or chemically synthesized, may be chosen from genes encoding any protein which can be used as a therapeutic or cosmetic compound, or as a diagnostic reagent or as a compound which can be used in a biosynthesis or biodegradation process. The protein of interest can consist of a mature protein, a precursor, and in particular a precursor intended to be secreted and comprising a signal peptide, a truncated protein, a chimeric protein originating from the fusion of sequences of diverse origins, or a mutated protein having improved and/or modified biological properties.

The invention also comprises a process for producing a protein , characterized in that the culture medium of step a) also comprises the compounds required for inducing the synthesis of the protein encoded by said homologous or heterologous gene of interest. These compounds are known to those skilled in the art and depend, in particular, on the cell and on the homologous or heterologous gene selected.

In any method to diversify the chemical composition of proteins produced in vivo comprising the step of disabling the editing function of an aminoacyl tRNA synthetase, it is preferred that said aminoacyl tRNA synthetase is an aminoacyl tRNA synthetase selected from the group consisting of the aminoacyl tRNA synthetase comprising an editing function corresponding to an editing site or domain encoded by a portion of the DNA encoded said aminoacyl tRNA synthetase, preferably encoded by a DNA portion having at least conserved residues compared after alignment with the editing site of the valyl-tRNA synthetase and isoleucyl-tRNA synthetase as shown in figure 2A, preferably selected from the group consisting of the aminoacyl tRNA synthetase valyl-tRNA synthetase, isoleucyl-tRNA synthetase, leucyl-tRNA synthetase, alanyl-tRNA synthetase, prolyl-tRNA synthetase, threonyl-tRNA synthetase, phenyl-tRNA synthetase and lysyl-tRNA synthetase which are known to have an editing site or domain (see for Ile RS Baldwin, A. N. and Berg, P. (1966) J. Biol. Chem. 241, 839-845 and.Eldred, E.W. and Schimmel, P. R. (1972) J. Biol. Chem. 247, 2961-2964; for Val RS, Fersht, A. R. and Kaethner, M. M. (1976) Biochemistry. 15 (15), 3342-3346; for Leu RS, English, S. et al., (1986) Nucleic Acids Research. 14 (19), 7529-7539; for Ala RS, Tsui, W. -C. and Fersht, A. R. (1981) Nucleic Acids Research. 9, 7529-7539; for Pro RS, Beuning, P. J. and Musier-Forsyth, K. (2000) PNAS. 97 (16), 8916-8920; for Thr RS, Sankaranarayanan, R. et al., (2000) Nat. Struct. Biol. 7, 461-465 and Musier-Foryth, K. and Beuning, P.J. (2000) Nat. Struct. Biol. 7, 435-436; for PheRS, Yarus, M. (1972) PNAS. 69, 1915-1919 and for LysRS, Jakubowski, H. (1997) Biochemistry. 36, 11077-11085.

### EXAMPLES

### Example 1

A direct selection for restoring an enzymatic activity through incorporation of Abu could not be easily set up because its aliphatic side chain lacks chemical reactivity and therefore cannot act as a catalytic residue. We thus resorted to an indirect scheme based on the structural resemblance of Abu with Cys (Figure 1A), an essential catalytic residue in numerous enzymes. Selecting a synthetase mutant that mischarged its cognate tRNA with Cys might result in Abu being mischarged by the mutant synthetase.

We took advantage of the *thyA* conditional selection screen in *E. coli*, based on the absolute requirement for an active thymidylate synthase when thymidine is not provided as a growth factor (13). This same screen was used previously to assess the potency of suppressor Cys-tRNAs in codon misreading (14) and to enforce phenotypic suppression by the non-canonical azaleucine (15). An entire set of plasmid-borne *thyA* alleles with all 64 different codons at position 146 was constructed for altering the catalytic site occupied by an essential Cys (16, 17). Each allele was tested for its ability to restore growth to an *E. coli* strain (lacking the chromosomal copy of *thyA*) on mineral glucose medium in the absence of thymidine (14, 15). Three of the 64 plasmid-borne *thyA* alleles restored growth. These had one of three codons-UGU, UGC, or UGA. The growth responses of the UGU and UGC alleles were expected, as these code for cysteine. The positive response of UGA (a termination codon in *E. coli*) likely results from read-through by Cys-tRNA, and thus demonstrates the sensitivity of the selection assay.

Strains bearing inactive alleles of *thyA* were then tested to see if they could be suppressed by supplying them with excess L-cysteine in mineral medium devoid of thymidine. Shallow growth was reproducibly observed on L-cysteine gradient plates (18) for the missense alleles having any of the eight codons AUN and GUN alone. Growth was stronger with alleles bearing any of the four Val codons (GUU, GUC, GUA, and GUG) than for those with the three Ile codons (AUU, AUC, and AUA) or for the Met codon AUG. Cysteine-suppression of the three Ile codon-bearing alleles and of the Met codon-bearing allele was abolished by addition of exogenous L-isoleucine and L-methionine, respectively. Suppression of the four Val codon-bearing alleles was abolished by addition of exogenous L-valine plus L-isoleucine but not by L-isoleucine alone (18). The four Val146 alleles gave a similar growth response in cysteine gradient plates, despite being decoded by three different tRNA^{Val} isoacceptors, thus suggesting that Cys is being mischarged onto all three Val isoacceptor tRNAs by ValRS. Altogether, these results suggested that ValRS catalyzed the formation of Cys-tRNA^{Val} *in vivo* at a rate sufficient for active thymidylate synthase production and that this mischarging reaction was prevented by increasing the intracellular concentration of L-valine. This interpretation is in line with earlier reports of Cys misactivation by ValRS *in vitro* (11).

Suppression of *thyA*:*Val146* alleles was weak on plates and required high L-cysteine concentrations (developed from a gradient starting at a concentration of 100 mM). It could thus be anticipated that a scarce L-cysteine supply should select for an enhanced efficiency of phenotypic suppression. Two experimental procedures were followed to this end. In the first procedure strain β5519 *(thyA:: erm*+ *ΔnrdD::kan*+ pTS13 (*bla*+ *thyA*:146GUA) was propagated over 100 generations in serial liquid culture with limiting cysteine (1.5 mM) under anaerobic conditions resulting in isolation of strain β5420 (19). The second procedure relied on a one-step selection under aerobic conditions on plates containing a non-oxidizable precursor that is inefficiently converted into cysteine by *E.coli* (S-carbamoyl-cysteine (Scc, FigurelA) (19)). A mutator marker (*dnaQ* or *mutS*) was introduced into the test strain β5519 to increase the frequency above 10⁻¹⁰ of Scc-suppressible thymidine auxotrophs (19). This approach resulted in the isolation of four strains: β5456, β5479, β5485 and β5486. All five isolated strains were found not to grow at 42, in agreement with the heat sensitivity generally caused by translational errors (20). The cysteine-suppression phenotype is shown for one of these strains (β5456) in Figure1B, together with its abolition by L-valine (supplied as an Ile-Val peptide).

Judging from these phenotypes, mutations in the *valS* gene for valyl-tRNA synthetase were suspected for each isolated strain. To test for this possibility, we took advantage of the *nrdD::kan*+ marker (19) located 0.4 min from *valS* on the *E. coli* chromosome. For the five mutants, the trait of cysteine- or Scc-suppressible thymidine auxotrophy was indeed found to co-transduce with the *nrdD::kan*+ marker in a proportion of about 45 %. Further characterization of the *valS* mutations was performed by PCR-amplification followed by sequencing of five *nrdD::kan*+ transductants exhibiting Scc-suppressible thymidine auxotrophy derived from the five isolated strains (21). As shown in Table 1, each of the five mutants contained a single amino acid substitution at positions within the conserved editing domain (known as CP1) of ValRS (22). The following mutations were identified: T222P, R223H, D230N, V276A, and K277Q. Remarkably, two of these positions (T222 and D230) align with conserved positions in IleRS that had been demonstrated previously to be involved in the hydrolytic editing of misacylated Val-tRNA^{lle} (23) (Figure 2A).

**Table 1. Mutations of the valS gene selected by suppression of the thymidine auxotrophy of strain β5419 (ΔthyA::erm⁺ ΔnrdD::kan⁺pTS13 (bla⁺ thyA:146GUA)).**

| Strain | Method of isolation | Mutator genotype | Codon of *valS* | | | | |
|---|---|---|---|---|---|---|---|
| | | | **222** | **223** | **230** | **276** | **277** |
| β5419 | | wt | **ACC** | **CGT** | **GAT** | **GTG** | **AAA** |
| | - | | **Thr** | **Arg** | **Asp** | **Val** | **Lys** |
| β5456 | Selection on Scc | *ΔdnaQ* | **CCC Pro** | CGT | GAT | GTG | AAA |
| β5479 | Selection on Scc | *ΔmutS* | ACC | **CAT His** | GAT | GTG | AAA |
| β5486 | Selection on Scc | *ΔmutS* | ACC | CGT | **AAT Asn** | GTG | AAA |
| β5485 | Selection on Scc | *Δmuts* | ACC | CGT | GAT | **GCG Ala** | AAA |
| | Selection | | | | | | **CAA** |
| β5520 | on Cys under anaerobiosis | wt | ACC | CGT | GAT | GTG | **Gln** |

VaIRS is known to misactivate Thr and generate Thr-tRNA^{Val}, which normally is hydrolyzed by the ValRS editing activity (9). If the VaIRS mutants in Table 1 are impaired for editing, then strains bearing each of these mutations should misincorporate Thr into protein, and L-threonine would then be toxic in these strains. The growth of the five different *valS* strains was therefore tested in the presence of L-threonine. All displayed high sensitivity towards exogenous L-threonine (at 2 mM), while the parent strain β5419 was insensitive to L-threonine at all concentrations. The results for the strain carrying the *valS:T222P* allele are shown in Figure 1C.

The phenotype of the *valS:T222P* allele suggested that the T222P enzyme mischarged tRNA^{Val} with Thr and Cys *in vivo.* The T222P mutant enzyme was, therefore, expressed and partially purified so that it could be directly assayed for the ability to misacylate tRNA^{Val}. The purified enzyme had the same activity as the wild-type enzyme for charging with valine (Figure 2B, inset). In contrast, the T222P mutant enzyme misacylated tRNA^{Val} with Thr to give Thr-tRNA^{Val} while the wild-type enzyme produced no detectable mischarged tRNA^{Val} (Figure 2B). Misacylation of tRNA^{Val} with cysteine was also catalyzed only by the mutant enzyme.

We anticipated that VaIRS mutants that misincorporated Cys would also misincorporate Abu. Indeed, the strain carrying the *valS:T222P* allele on the chromosome (β5456) was sensitive to Abu (Figure 1C) (whereas its wild-type counterpart was not), suggesting incorporation of Abu in response to Val codons. With this in mind, we shewed that Abu could contribute to the relief of L-valine auxotrophy of the *Δilv* strain CU505, but only in the presence of the *valS:T222P* allele (19). Whole cell protein was isolated from strain CU505 grown in the presence of Abu (0.2 mM), in the presence or absence of the *valS:T222P* allele in the host cell. Analysis of amino acid composition showed that 24 % of the valine was replaced by Abu only in the strain harboring the mutant allele (Table 2). Finally, the valine-rich yeast protein AlaXp (swissprot:P53960) was overexpressed and purified from strains containing the *valS:T222P* allele grown in the presence of Abu. The protein samples were digested by trypsin and analyzed by mass spectrometry. MALDI analysis showed that, when AlaXp was produced in the strain carrying the T222P mutation, it contained a mixture of Val and misincorporated Abu (Figure 2C). For a given peptide the degree of misincorporation ranged between 9.5 % and 18 % per Val codon. Sequencing of several Abu-containing peptides confirmed that Abu was specifically misincorporated into positions designated by Val codons.

**Table 2.** Incorporation of Abu into cells bearing wild-type and T222P mutant alleles of *valS.* Cultures of the *Δilv* strain CU505 (19) and the isogenic strain β35498 carrying the *valS:T222P* allele were grown overnight in minimal medium (27) with Ile-Leu (0.3 mM) and limiting valine (0.04 mM Ile-Val), diluted (1/2), and adjusted with Ile-Val (0.02 mM) with or without Abu (0.2 mM). After 24 hours of growth, total protein was extracted as follows. Cells were first harvested by centrifugation and washed in cold 10 % trichloroacetic acid (TCA, 1/2 of the culture volume). Cells were then re-centrifuged at 4000×g for 10 min, resuspended in cold 10% TCA (1/10 of the culture volume) and centrifuged again. The washed cells were resuspended in 5 % TCA, heated at 95 °C for 30 minutes and centrifuged (4000× g, 10 min). The precipitate was washed three times with cold acetone and dissolved in 50 mM NH₄HCO₃. Proteins were hydrolyzed in 6 N HCl-0.2% phenol at 110°C for 20 h in sealed tubes. Norleucine was added as an internal standard. Aliquots of the hydrolysates were analyzed on a Beckman 6300 Amino Acid Analyzer. Amino acids were quantified by appropriate standards and values are presented relative the wild-type (WT) control that lacked Abu.

| Amino acid incorporated | WT | T222P | WT + Abu | T222P+ Abu |
|---|---|---|---|---|
| Abu | 0.0 | 0.0 | 0.0 | 0.24 |
| Val | 1.0 | 0.95 | 1.0 | 0.73 |
| Val+Abu | 1.0 | 0.95 | 1.0 | 0.97 |
| Ile | 1.0 | 1.0 | 1.0 | 1.0 |
| Ala | 1.0 | 0.97 | 1.0 | 0.92 |

### Example 2

**Directed evolution scheme.** To avoid a decrease of cysteine concentration in the medium due to oxidation, the selections were carried out under strictly anaerobic conditions. The biomass of cells in cultures relative to the cysteine concentration (measured as optical density at 600 nm after 24 h of growth at 30 °C) gradually increased four-fold when the *thyA* strain β5366 (*ΔthyA::erm*⁺ pTS13 (*bla*⁺ *thyA*:146GUA)) was propagated by serial transfer in mineral glucose medium supplemented with 1.5 mM cysteine. A high concentration of cysteine was required because the input population could hardly be propagated otherwise. After 16 inoculations, each at a dilution of 1/100 (a total of about 100 generations), single colonies were isolated on mineral glucose plates supplemented with thymidine (a representative of which was designated strain β5520).

**One-step selection scheme.** For the one-step selections, cysteine oxidation under aerobiosis and subsequent cystine precipitation from the growth medium was avoided by use of the non-oxidizable precursor *S*-carbamoyl-cysteine (Scc). Scc is a poor precursor of cysteine and sustains growth of a Cys-auxotrophic *E. coli* strain less efficiently than cysteine. However, at concentrations above 1 mM, Scc gave rise to suppression of the thymidine auxotrophy of strain β5520, while in the absence of thymidine no growth was detectable below 1 mM Scc. When β5419 cells bearing the *thyA:Val146GUA* allele on a high copy plasmid were plated on mineral glucose plates supplemented with Scc (3 mM), no colonies grew after prolonged incubation at 30°C. (The experiment was designed to detect a mutant at a frequency of 10⁻¹⁰.) To increase the mutation frequency, a *mutS::spc*+ (33) mutator allele was introduced in the genetic background of strain β5419 by P1 transduction, yielding strain β5432 (the *mutS::spc*+ disruption disables the mismatch repair system and leads to random transitions and frameshifts (34)). Colonies then appeared on the same medium with a frequency of about 10⁻⁸. No colonies were found on plates lacking Scc. A comparable frequency of Scc-suppressible clones was obtained after introduction by P1 transduction (33) of a *dnaQ* mutator allele (33) (to give strain β5435).

**Abu misincorporation.** The valine auxotroph CU505 (33) was grown in the presence of a limiting supply of valine and increasing concentrations of Abu (Figure 3). The biomass of cells in cultures relative to the valine concentration in the medium did not change up to a 1 mM concentration of Abu. In contrast, when the *valST222P* allele was introduced into the chromosome (strain β5498), the yield of cells was diminished in the absence of Abu but increased up to 30% when Abu (0.2 mM) was added.

Thus, *E. coli* strains that proliferate only because of infiltration of the Val coding pathway were selected and all contained mutations leading to single amino acid substitutions in the editing site of ValRS. This observation is consistent with a central role for editing in restricting the genetic code to twenty amino acids, by preventing the invasion of other amino acids such as Abu. Indeed, the editing sites in IleRS and VaIRS are rigorously conserved in even the most deeply branched organisms in the tree of life. However the translation accuracy maintained by editing may prevent further chemical diversification of proteins. Thus, disabling the editing function of a synthetase, as demonstrated in the present work, offers a powerful approach to diversify the chemical composition of proteins produced *in vivo.*

For some synthetases, accuracy depends critically on an editing function at a site distinct from the aminoacylation site. Mutants of *Escherichia coli* that mischarge tRNA^{Val} with cysteine were sought by random mutagenesis of the whole chromosome. All mutations obtained were located in the editing site of valyl-tRNA synthetase. Over 20 % of the valine in cellular proteins from such an editing mutant organism could be replaced with the noncanonical aminobutyrate, sterically similar to cysteine. Thus, the editing function may have played a central role in restricting the genetic code to twenty amino acids. Disabling this editing function offers a powerful new approach for diversifying the chemical composition of proteins and for emulating ambiguous evolutionary stages of translation.

### Example 3: Correlation between toxicity of α-aminobutyrate for E. coli strains harboring different valS alleles mutated in the editing domain and results of in vitro editing assays carried out with the corresponding ValRS enzyme variants

To investigate more deeply the editing phenotypes of the five aforementioned mutant VaIRSs, and the relationship between cell viability and editing, plasmids harboring genes for mutant and wild-type ValRSs were constructed and placed under control of an arabinose-inducible promoter. These constructs were then used to investigate the *in vivo* phenotypes of the mutant enzymes. Separately, His-tagged versions of the mutant enzymes were constructed in parallel for use in purifying the enzymes for studies of aminoacylation and editing activities *in vitro.*

**Construction of valS knockout strains :** A 1.7kb portion of the valS gene from pET16valS that contained the region encoding the editing domain of VaIRS was excised using Sall and XhoI. This region was then replaced with a kanamycin marker (1.2kb in size) liberated from pUC4K by flanking Sall restriction sites. Overhanging regions of the valS gene of 406 and 708 base pairs respectively were left to allow for homologous recombination (resulting plasmid named pLAN362). The kanamycin marker contained its own promoter and was inserted in the forward orientation with respect to the valS gene. To increase the amount of linearized product used for homologous recombination, the valS::kan from pLAN362 was PCR-amplified using primers annealing to the T7 promoter and terminator. The products of this reaction were visualized and purified in a 1% agarose TAE gel. To get recombination into the E. coli chromosome, the linearized valS::kan was transformed by electroporation into a hyper recombinant strain of E. coli (JC8679, Stewart et al.) containing plasmid-borne valS from Haemophilus influenzae (pSU18valS). Recombinants were selected on Luria Broth agar plates supplemented with chloramphenicol (strain JC8679 is CmR) and low kanamycin (25ug/ml) because efficiency of kanR markers can be decreased when it recombines into the chromosome, one recombinant chosen and named strain PS2838. PS2838 was infected with a P1 phage stock, phage were allowed to propagate for 3 hours then were stored in CHC13 at 4C. Transfer of genes and resistance markers was done by P I phage transduction using standard protocols (Miller 1972) into MG1655 wild-type E. coli containing each of the valSpBAD constructs expressing either wild-type VaIRS, T222P, R223H, D230N, V276A, and K277Q. Transductants were selected by plating on Luria Broth agar plates supplemented with kanamycin (25ug/ml) and 0.2% arabinose, incubating at 25C for 48 hours. As expected transduction of the MG1655 strain alone did not yield colony growth. All transductants were restreaked onto media supplemented with 50ug/ml kanamycin to ensure kanR and insertion of kanamycin marker into the E. coli chromosomal valS gene was verified by amplifying with a forward primer that annealed to a region on the chromosome just upstream of the valS gene (valS.103) and a reverse primer annealing to the marker itself (puc4k.ol4). Transduction yielded the following strains of valS::kanR: wt VaIRS-PS2847, T222P-PS2849, R223H-PS2862, D230N-PS2865, V276A-PS2851, K277Q-PS2853.

**Plasmid Construction:** A cautious approach was taken in this construction due to a lack of success introducing valS editing mutants previously. With this in mind initial constructs were done in a variation of pBAD18, a plasmid allowing for tight regulation of expression under control of an arabinose inducible promoter. Parent plasmid was a Histidine tagged valS construction received from Jack Horowitz which we verified by sequence analysis to done be pET16b (Novagen). Utilizing an internal restriction site and a restriction site located downstream of the valS gene in the multiple cloning site of pET16b, a large *BamHI* fragment was subcloned into pUC19 (pSCO2). In parallel, the parent plasmid was digested with *SalI* and *BglII*, restriction sites that flank the region of valS encoding the CPI, and this small segment was ligated into pUC19 (pSCO5). The QuikChange mutagenesis kit (Stratagene, La Jolla, CA) was used on pSCO5 to introduce an A to C base change at nucleotide 663 to achieve the Thr to Pro substitution in residue 222 of ValRS. Both the wild-type and T222P *SalI-BglII* fragments were subcloned into pSCO2. The resulting vectors were digested with *NcoI* and *SmaI* to release fragments of the entire downstream region of valS which were then cloned into pVDC441, a variation of pBAD18 into which the upstream portion of valS from the pET16bvalS plasmid had been cloned using *EcoRI* and *KpnI.* The His tagged T222P construct was constructed by subcloning the mutated region of valS (*DraIII*/*XhoI*) from pVDC447 and ligating it into the pET16bvalS. The wild-type valS constructs, pBAD18valS and pET16bvalS, were eventually used as templates in the QuickChange mutagenesis protocol to generate mutations in valS corresponding to the mutants R223H, D230N, V276A, and K277Q. The entire valS gene for these vectors was then sequenced for each of these vectors to verify their integrity.

**Mutant Toxicity Response to High Levels of α-Aminobutyrate:** ΔvalS::kan+ strains expressing wild-type ValRS, T222P, R223H, D230N, V276A, and K277Q were each isolated on mineral standard medium (MS) (Richaud et al, 1993) supplemented with 0.2 % glycerol, 0.02 % arabinose and ampicillin(100ug/mL). Single colonies from each strain were inoculated into liquid cultures of the same media composition and grown overnight to saturation. Cells were diluted in media 1:100 and a lawn of cells was spread and allowed to dry onto MS medium plates. α-aminobutyrate (50uL of 0.1M) was added to a central well created in each plate and plates were then incubated for 24 hours at 42C. The relative response of each strain to high levels of noncognate α-aminobutyrate could then be evaluated based on diameter of toxicity halo observed.

**Expression and Purification of ValRS Proteins:** pET16b plasmids corresponding to the wild-type VaIRS and each of the ValRS mutants were transformed into BL21 competent cells (Novagen). These cells were cultured in 250mL of Luria Broth supplemented with ampicillin (100ug/mL), when cells reached an optical density at 600nm of 1.0 expression of VaIRS was induced with 1mM IPTG for 5 hours. Cells were then stored at -80C until purification. Cells were resuspended (50mM Na₂PO₄, 300mM NaCI, 50mM B-ME, 30mM Imidazole pH 7.4) and lysed 2x in French Press. Lysates were bound a Ni-NTA affinity column and eluted in lysis buffer with a gradient ranging from 30mM to 250mM Imidazole. Collected fractions were visualized on an 8 % SDS-polyacyrlamide gel stained with coomassie brilliant blue to ensure purity, purest fractions were pooled and dialyzed into 25mM Tris-HCl, 1mM B-ME pH 7.5. Enzyme concentrations were determined by Bradford assay.

**Aminoaclyation Assay, Misacylation, and Deacylation :** Aminoacylation assays were performed at 37C in a 100uL volume containing buffer (20mM HEPES, 0.1mM EDTA disodium salt, 0.15M NH₄Cl, 10ug/mL BSA pH 7.5), 2µM MgCl₂, 0.7µM [³H]Val, 20µM cold Val, 2µM tRNAᵥₐₗ (Sigma) and 20nM VaIRS enzyme (adapted from Hendrickson et al. 2000). Aliquots (10µL) of the reaction mixture were precipitated with tricholoracetic acid, and the level of aminoacylation of the tRNA was determined by scintillation counting. Misacylation of Thr onto tRNAᵥₐₗ was performed in the same conditions except 5.86µM [³H]Thr was substituted forVal in the reaction. To test deacylation rates for these enzymes it was necessary to generate [³H]Thr-tRNAᵥₐₗ. This was done using ValRS with the T222P substitution purified previously (Doring et al, 2001), this enzyme was shown to form the [³H]Thr-tRNAᵥₐₗ complex. Enzyme was incubated with 2.5µM tRNAᵥₐₗ and 45uM [³H]Thr, incubated at 37C for 45 minutes, extracted twice in phenol:chloroform, and ethanol precipitated. Pellet was dissolved in 100µL sterile H₂O and scintillation counting was used to determine the success of the reaction. Deacylation reactions, done in triplicate for each enzyme, were performed in 150mM Tris-HCl (pH7.5), 100µg/ml BSA, 10mM MgCl2. At room temperature 2nM enzyme was combined with [³H]Thr-tRNAᵥₐₗ, at 3, 6, 9, 16, and 30 minutes aliquots (9µL) of the reaction mixture were precipitated with tricholoracetic acid, and the amount of misaminoacylated [³H]Thr-tRNAᵥₐₗ remaining in the sample was determined by scintillation counting A no enzyme control reaction was included to provide reference point.

When the percentage of Thr-tRNA^{Val} hydrolyzed by each enzyme in deacylation assays *in vitro* was compared to the observed Abu-induced inhibition zone diameters *in vivo,* a pattern was clear (Figure 5). The T222P and K277Q VaIRS mutant proteins have the most severe defects, for example, in deacylation ofThr tRNA^{Val} *in vitro.* Strains bearing these mutations show a toxic response to only slightly raised levels of Abu *in vivo.* These two enzymes also accumulated high levels of mischarged Thr-tRNA^{Val} *in vitro.* The D230N mutation appears to be less detrimental to the deacylase activity, exhibiting a slowed rate of deacylation. Similarly, toxicity *in vivo* was limited to intermediate levels of Abu. Having the V276A mutation appears to impart the least toxic response to exogenous Abu. This phenotype is reflected in a deacylation rate that is closer to wild-type VaIRS than to that of the other mutant enzymes. Both D230N and V276A ValRS showed low levels of mischarging with respect to the other mutant enzymes as well. Thus, the ability of the mutant enzymes to hydrolyze *in vitro* misaminoacylated amino acids from tRNA^{Val} correlated with the toxicity *in vivo* of exogenous noncognate amino acids that were added to cells bearing the same mutations in their VaIRS.

### Example 4: Construction of strains of E. coli expressing an isoleucyl-tRNA synthetase mutated in the editing site

Several studies have identified the editing domain of IleRS (Schmidt at Schimmel, 1004, Science), and more particularly the amino acids of the 240 to 250 region which are extremely conserved in the IleRS sequences of different organisms. Point mutations in this region generate enzymes partially or totally deficient in the editing function (Hendrickson, 2000). An artificial allele of the gene *ileS* coding for isoleucyl-tRNA E. coli synthetase containing a succession fine residues replacing residues 240 to 250 has been constructed in the following manner: the the pVDC433 (Hendrickson et al. 2001), derived from plasmid pBAD (Guzman et al., 1995) by insertion of the wild type ileS gene is digested by restriction enzymes *Cla*I and *Spe*I according to the instructions of the supplier, thus eliminating nucleotides 717 to 750 of the gene. The 5' phosphorylated oligonucleotides (Invitrogen Life Technologies):
5'pCTAGTAATCGCGGCGGCGGCGGCGGCGGCGGCGGCGGCGGCGCGCGCAATAT and
5'pCGATATTGCGCGCGCCGCCGCCGCCGCCGCCGCCGCCGCCGCCGCGATTA
are hybridized and ligated with the plasmid pVDC433, previously cut by *Sla*I and *Spe*I under the following conditions: 0.3 pmoles of vector and 3 pmoles of each oligonucleotide are incubated for 10 minutes at 70 degrees C after precipitation and resuspension in 10 ul of H₂O, after return to room temperature; the ligation is carried out using standard protocols. The ligation mixture is utilized to transform competent cells of the SureTM strain (Stratagene) by electroporation following the protocol of the supplier. The transformants are obtained and plasmid DNA prepared (Qiaprep Kit, Qiagen). The plasmid obtained in this manner, pTLH33, is sequenced and the insertion of 11 alanine codons is verified.

The pTLH33 plasmid is inserted in the wild type *E. coli* strain MG1655 to obtain strain PS2419. The allele obtained by deletion of the ileS gene, deltaileS203::kan, from strain IQ839 (Shiba and Schimmel, 1992) is introduced in strain PS2419 by P1 transduction according to a standard protocol (Miller, 1972), and a transducant, strain PS 2449, is obtained in rich medium supplemented with kanamycin (25 mg/L) and arabinose (0.02 %). In the same way, strain PS2306 is constructed by insertion of the pVDC433 plasmid in strain MG1655 (resulting strain: PS27752) and P1 transduction of allele delta ileS203::kan in strain PS2752. Growth of the two strains deleted in the ileS locus, PS2306 and PS2449, requires the presence of arabinose, thus demonstrating the controlled expression of the wild type ileS gene (plasmid PVDC433, strain PS2306) and the mutated gene iles ala11 (plasmid pTLH33, strain PS2449) respectively.

### Exemple 5 : Sensitivity of the isoleucyl-tRNA synthetase to non canonic amino acids

Strains PS2306 and PS2449 are tested for their sensitivity to artificial amino acids whic present a steric resemblance to isoleucine. The cells are cultivated in MS mineral medium, succinate (0.2%), arabinose (0.2%) for 24 h at 37 degrees centigrade and diluted at 1/250 in MS mineral medium. 0.5 ml of this cell suspension are spread on a Petri dish containing 25 ml of MS medium supplemented with succinate (0.2%) and arabinose (0.2%). A well is then prepared in the middle of the dish and filled with 0.1 ml of an amino acid solution:
1) 25 mM S-methyl cysteine
2) 25 mM homocysteine
3) 25 mM o--methyl-L-serine
4) 25 mM Norleucine
5) 25 mM Norvaline

The Petri dishes are then incubated at 37 degrees centigrade for 24 hours and the appearance of a zone of inhibition around the well is recorded. The diameters of the zones of attenuated growth are measured as follows:

**Table 3.**

| Acide aminé | PS2306 | PS2449 |
|---|---|---|
| S-methyl-L-cystéine | 1,3 cm | 3,0 cm |
| Homocystéine | 2,1 cm | 2,9 cm |
| O-methyl-L-sérine | 2,2 cm | 5,6 cm |
| Norleucin | 2,9 cm | 3,4 cm |
| Norvaline | 3,7 cm | 8,0 cm |

All 5 non-canonic amino acids tested inhibit the growth of the two strains, but it is noted that in all cases there is a stronger inhibition on the strain expressing the mutated allele of the *ileS* gene in the editing site. Thus the mutated isoleucyl-tRNA synthetase seems to have an increased specificity for the substrate amino acid capable of loading tRNAile with non-natural amino acids.

### Example 6: Biochemical characterization of the IleRS240-250Ala mutant

The wild type and mutant IleRS enzymes were purified from strains PS2306 and PS2449 by chromatography using the procedure described by Hendrickson et al. (2002). Ile-tRNA loaced with the amino acid Val (Val-t-RNAlle) can be produced in large quantity by utilizing the editing mutant IleRS T242P (Hendrickson 2000). The utilzation of this Val-tRNA/Ile has allowed testing of deacylation activity of several enzymes using the protocol described by Hendrickson, 2000. The results obtained, shown in figure 4, clearly show that the IleRS240-250 mutant is deficient in the deacylation function of Val-tRNAIle.

### References and notes

1. U. L. RajBhandary, *J. Bacteriol.* **176,** 547-52 (1994).
2. A. Böck, et al., *Mol. Microbiol.* **5,** 515-20 (1991).
3. A. L. Weber, S. L. Miller, *J. Mol. Evol.* **17,** 273-84 (1981).
4. I. G. Fotheringham, N. Grinter, D. P. Pantaleone, R. F. Senkpeil, P. P. Taylor, *Biorg. Med Chem.* **7,**2209-13 (1999).
5. M. Ibba, D. Söll, *Annu. Rev. Biochem.* **69,** 617-50 (2000).
6. R. B. Loftfield, D. Vanderjagt, *Biochem. J.* **128,** 1353-1356 (1972).
7. A.N. Baldwin, P. Berg, *J. Biol. Chem.* **241,** 839 845 (1966).
8. E. W. Eldred, P. R. Schimmel, *J. Biol. Chem.* **247,** 2961-4 (1972).
9. A. Fersht, in *Structure and mechanism in Protein Science.* (Freeman, New York, 1999) pp. 389-399.
10. K. Musier-Forsyth, P. J. Beuning, *Nat. Struct. Biol.* **7**, 435-436 (2000).
11. H. Jakubowski, A. R. Fersht, *Nucleic Acids Res.* **9**, 3105-17 (1981).
12. D. R. Liu, P. G. Schultz, *Proc. Natl. Acad. Sci. U.S.A.* **96,** 4780-5 (1999).
13. M. Belfort, G. Maley, J. Pedersen-Lane, F. Maley, *Proc. Natl. Acad. Sci. U.S.A.* **80**, 4914-8 (1983).
14. V. Döring, P. Marlière, *Genetics* **150,** 543-51 (1998).
15. B. Lemeignan, P. Sonigo, P. Marlière, *J. Mol. Biol.* **231**, 161-6 (1993).
16. I. K. Dev, B. B. Yates, J. Leong, W. S. Dallas, *Proc. Natl. Acad. Sci. U.S.A* **85,** 1472-6 (1988).
17. The 64 alleles of *thyA* with different codons at position 146 of the coding sequence were constructed as follows. First, a unique *Nhe*I site was introduced through a G429→A substitution in the *thyA* coding region by site-directed mutagenesis (24) of plasmid pTSO (14) to yield plasmid pTSO1. Oligonucleotides THY1 (5'-CTGGATAAAATGGCGCTAGCACCGTGCCATGCATTC-3') and THY2 (5'-TCTGCCACATAGAACTGGAAGAATGCATGGCACGGT-3') were used for this mutation which preserved the sense of the codon thus mutated. Plasmid pTSO1 was then digested with *Nhe*I and *Nsi*I to remove from the *thyA* coding region an 18 bp fragment containing codon 146 (UGC). All 64 oligonucleotides of the *thyA* coding sequence from nucleotides 427 to 444 and the 64 oligonucleotides of the partial reverse sequence were constructed (GENAXIS Biotechnology, Montigny le Bretonneux, France). The 64 pairs of complementary oligonucleotides were annealed and ligated with the digested plasmid pTSO1.
18. Cysteine gradient plates were done as described in Fig. 1B legend. The effects of L-valine alone could not be directly examined because exogenous L-valine is known to inhibit growth of *E. coli* K12 in minimal medium (25). This inhibition is relieved if L-isoleucine is also supplied. Thus, the Ile-Val dipeptide was used as a valine source, because this dipeptide is transported across the cell membrane and then broken down to isoleucine and valine (26).
19. V. Döring, et al., Supplementary data can be found at the Science Web site.
20. M. J. Pine, *Antimicrobio. Agents Chemother.* **13,** 676-85 (1978).
21. *E. coli* chromosomal DNA was extracted using a DNeasy Tissue Kit (Qiagen GmbH, Hilden, Germany) following the instructions of the manufacturer. PCR to amplify the *valS* gene was performed as follows: denaturation at 94°C for 3 min, followed by 30 cycles of 30 sec at 94°C, annealing at 57°C for 30 s, and primer extension at 72°C for 200 sec. The final step was a primer extension at 72°C for 600 sec. The reaction was carried out using 2 units of Vent DNA polymerase (New England Biolabs, Beverly, MA) and 100 ng of chromosomal DNA in a 100 µl reaction mixture. The following primers were used: VAL1 (5'-GGGGAATTCGGTGTGTGAAATTGCCGCAGAACG-3'), and VAL2 (5'-GGCAAGCTTTCAGGTATTTGCTGCCCAGATCGA-3'). Two independent PCR amplification products of each mutant were sequenced (GENAXIS Biotechnology, Montigny le Bretonneux, France).
22. L. Lin, S. P. Hale, P. Schimmel, *Nature* **384,** 33-4 (1996).
23. O. Nureki, et al., *Science* **280,** 578-82 (1998).
24. M. Ansaldi, M. Lepelletier, V. Mejean, *Anal. Biochem.* **234,** 110-1 (1996).
25. M. De Felice, et al., *J. Mol. Biol.* **156,** 1-7 (1977).
26. A. J. Sussman, C. Gilvarg, *Annu. Rev. Biochem.* **40,** 397-408 (1971).
27. C. Richaud, et al., *J. Biol. Chem.* **268,** 26827-35 (1993).
28. E. Schmidt, P. Schimmel, *Biochemistry* **34,** 11204-10 (1995).
29. L. Lin, P. Schimmel, *Biochemistry* **35,** 5596-601 (1996).
30. L. M. Guzman, D. Belin, M. J. Carson, J. Beckwith, *J. Bacteriol.* **177**, 4121-30 (1995).
31. T. L. Hendrickson, T. K. Nomanbhoy, P. Schimmel, *Biochemistry* **39,** 8180-8186 (2000).
32. K. Gevaert, J. Vandekerckhove, *Electrophoresis* **21,** 1145-54 (2000).
33. *mutS*::*spc*+ allele (gift of F. Taddei, Hôpital Necker-Enfants, Paris); *ΔnrdD*::*kan*+ allele, laboratory collection; *ΔdnaQ*::*tet* allele (gift of J. Shapiro, University of Chicago, IL); CU505 (Δ(*ilvE*-*ilvC*)2049 *leu*455 *galT*1 IN(*rrnD-rrnE*)1) gift of Dr. M. Berlyn from the *E. coli* Genetic Stock Center (New Haven, CT). Transfer of genes and of resistance markers by P1 transduction were carried out using standard protocols (35).
34. P. Modrich, *Annu. Rev. Genet.* **25,** 229-53 (1991).
35. J. H. Miller, *Experiments in Molecular Genetics* (Cold Spring Harbor Laboratory Press, Cold Spring Harbor, NY, 1972).
36. Guzman, L.M., Belin, D., Carson, M.J., and Beckwith, J. (1995). Tight regulation, modulation, and high-level expression by vectors containing the arabinose PBAD promoter. J. Bacteriol. 177, 4121-4130
37. Hendrickson, T.L., Nomanbhoy, T.K., and Schimmel, P. (2000). Errors from selective disruption of the editing center in a tRNA synthetase. Biochemistry 39, 8180-8186
38. Hendrickson, T.L., Nomanbhoy, T.K., de Crecy-Lagard, V., Fukai, S., Nureki, O., Yokoyama, S., and Schimmel, P. (2002). Mutational separation of two pathways for editing by a class I tRNA synthetase. Mol. Cell 9, 353-362
39. Schmidt, E., and Schimmel, P. (1994). Mutational isolation of a sieve for editing in a transfer RNA synthetase. Science 264, 265-267
40. Shiba, K., and Schimmel, P. (1992). Functional assembly of a randomly cleaved protein. Proc. Natl. Acad. Sci.USA 89, 9964-9968

## Claims

1. Isolated isoleucyl-tRNA synthetase variant, **characterized in that** the residues 240 to 250 of the editing domain of the protein encoded by the gene ile RS of *E.coli* are replaced by 11 successive alanine residues.

2. Isolated nucleic acid sequence encoding the isoleucyl-tRNA synthetase variant of claim 1.

3. Vector comprising a nucleic acid according to claim 2.

4. Isolated transformed cell comprising a nucleic acid according to claim 2 or a vector according to claim 3.

5. Method for the production of proteins comprising a noncanonical amino acid **characterized in that** said method comprises the following steps:
a) culturing an isolated transformed cell according to claim 4 in a culture medium comprising the noncanonical amino acid, or one of its precursors, capable of being mischarged by the cognate tRNA of the isoleucyl-tRNA synthetase variant contained in said cell; and
b) recovering the proteins comprising said noncanonical amino acid from the culture medium or from the cells of step a).

6. Method according to claim 5, **characterized in that** the culture medium of step a) comprises a non canonical amino acid selected from S-methyl-L-cystein, homocystein, O-methyl-L-serine, norleucin and norvalin.

## Patentansprüche

1. Isolierte Isoleucyl-tRNA-Synthetase Variante, **dadurch gekennzeichnet, dass** die Reste 240 bis 250 der Korrekturdomäne (editing domain) des durch das E. *coli* Gen ileRS kodierten Proteins durch 11 aufeinanderfolgende Alaninreste ersetzt sind.

2. Isolierte Nukleinsäuresequenz, die für die Isoleucyl-tRNA-Synthetase Variante gemäß Anspruch 1 kodiert.

3. Vektor, der eine Nukleinsäure gemäß Anspruch 2 umfasst.

4. Isolierte, transformierte Zelle, die eine Nukleinsäure gemäß Anspruch 2 oder einen Vektor gemäß Anspruch 3 umfasst.

5. Verfahren zur Herstellung von Proteinen, die eine nichtkanonische Aminosäure umfassen, **dadurch gekennzeichnet, dass** das Verfahren die folgenden Schritte umfasst:
a) das Kultivieren einer isolierten, transformierten Zelle gemäß Anspruch 4 in einem Kulturmedium, das die nicht-kanonische Aminosäure oder eine ihrer Vorstufen umfasst, welche durch die der in der genannten Zelle enthaltenen Isoleucyl-tRNA-Synthetase Variante zugehörigen tRNA fälschlicherweise übertragen werden kann;
b) und das Gewinnen der Proteine, die die genannte nicht-kanonische Aminosäure umfassen, aus dem Kulturmedium oder aus den Zellen aus Schritt a).

6. Das Verfahren gemäß Anspruch 5, **dadurch gekennzeichnet, dass** das Kulturmedium in Schritt a) eine nicht-kanonische Aminosäure ausgewählt aus S-Methyl-L-cystein, Homocystein, O-Methyl-L-serin, Norleucin und Norvalin umfasst.

## Revendications

1. Variant isolé d'isoleucyl-tRNA synthétase, **caractérisé en ce que** les résidus 240 à 250 du domaine d'édition de la protéine codée par le gène ile *RS* de *E*. *coli* sont remplacés par 11 résidus successifs d'alanine.

2. Séquence d'acide nucléique isolée codant pour le variant d'isoleucyl-tRNA synthétase selon la revendication 1.

3. Vecteur comprenant un acide nucléique selon la revendication 2.

4. Cellule transformée isolée comprenant un acide nucléique selon la revendication 2 ou un vecteur selon la revendication 3.

5. Procédé pour la production de protéines comprenant un aminoacide non canonique, **caractérisé en ce que** ledit procédé comprend les étapes suivantes :
a) culture d'une cellule transformée isolée selon la revendication 4 dans un milieu de culture comprenant l'aminoacide non canonique, ou l'un de ses précurseurs, capable d'être chargé de manière erronée par l'ARNt correspondant du variant d'isoleucyl-ARNt synthétase contenu dans ladite cellule; et
b) récupération des protéines comprenant ledit aminoacide non canonique à partir du milieu de culture ou à partir des cellules de l'étape a).

6. Procédé selon la revendication 5, **caractérisé en ce que** le milieu de culture de l'étape a) comprend un aminoacide non canonique choisi parmi la S-méthyl-L-cystéine, l'homocystéine, la O-méthyl-L-sérine, la norleucine et la norvaline.
